# DEMANDE DE BREVET EUROPEEN

(11) **EP 1 054 016 A1**
(43) Date de publication de la demande: **22.11.2000**
(21) Numéro de dépôt: 00401316.5
(22) Date de dépôt: 15.05.2000
(51) Int. Cl.: C07H 3/04

(54) **Préparation d'un mélange de glucosyl-mannitol et de glucosyl-sorbitol**

(30) Priorité: 17.05.1999 FR 9906226
(71) Demandeur: Roquette Frères, 62136 Lestrem (FR)
(72) Inventeur: Caboche, Jean-Jacques, 62131 Drouvin le Marais (FR)
(74) Mandataire: Boulinguiez, Didier

(57) **Abrégé**

L'invention concerne un procédé de préparation d'un mélange de glucosyl-mannitol et de glucosyl-sorbitol, caractérisé par le fait que l'on conduit l'hydrogénation de la glucosyl-glucosone en présence d'un catalyseur, et en soumettant une solution de glucosyl-glucosone présentant une matière sèche au moins égale à 10 % en poids, de préférence comprise entre 20 et 50 % en poids, à une pression au moins égale à 30 bars, de préférence comprise entre 30 et 200 bars, et à une température au moins égale à 50°C, de préférence comprise entre 50 et 150°C.

## Description

La présente invention est relative à un procédé de préparation d'un mélange de glucosyl-mannitol et de glucosyl-sorbitol par hydrogénation de la glucosyl-glucosone.

L'invention concerne en particulier un procédé de préparation d'un mélange de glucosyl-mannitol et de glucosyl-sorbitol par hydrogénation de la glucosyl-glucosone en présence d'un catalyseur, et en mettant en oeuvre, pour une matière sèche élevée en glucosyl-glucosone, des conditions de pression et de température d'hydrogénation particulières.

La présente invention concerne plus précisément un procédé de préparation d'un mélange de glucosyl-mannitol et de glucosyl-sorbitol par hydrogénation continue de la glucosyl-glucosone en lit fixe de catalyseur, et plus particulièrement la mise en oeuvre de ce procédé continu dans une succession de lits fixes de catalyseur disposés en série et en au moins deux zones de réaction.

La présente invention est enfin relative à un mélange de glucosyl-mannitol et de glucosyl-sorbitol, notamment un mélange d'isomères de liaison α-1,4 ou de liaison α-1,6 de glucosyl-mannitol et de glucosyl-sorbitol, contenant une proportion au moins équimolaire de glucosyl-mannitol et de glucosyl-sorbitol.

La préparation de ces mélanges d'isomères de liaison α-1,4 ou α-1,6 est classiquement réalisée soit par le mélange de chacun des deux composants en proportion souhaitée, soit par isomérisation ou épimérisation de disaccharides particuliers.

Ainsi, les glucosyl-α-1,4-mannitol et glucosyl-α-1,4-sorbitol (ou maltitol) peuvent être produits séparément et ensuite associés pour conduire au mélange attendu.

Cependant, si la préparation du maltitol est aisée, s'agissant de conduire l'hydrogénation d'un sirop de maltose obtenu à partir d'un hydrolysat d'amidon par tout moyen connu classiquement de l'homme du métier, la préparation du glucosyl-α-1,4-mannitol est réalisée par une étape d'isolement du sucre rare glycosyl-α-1,4-mannose à partir de milieux complexes (extraits d'algues ou de levures par exemple), par des procédés lourds et complexes avec de très faibles rendements, suivi d'une étape d'hydrogénation.

Quant aux isomères de liaison α-1,6, le mélange des deux composants glucosyl-mannitol et glucosyl-sorbitol correspondant est connu de l'homme du métier en proportion équimolaire sous le vocable de palatinitol.

Le palatinitol est un édulcorant de masse peu calorique et peu cariogène que l'on obtient par exemple par hydrogénation catalytique à pH neutre de l'isomaltulose ou glucosyl-α-1,6-fructose.

L'isomaltulose est lui-même obtenu par isomérisation enzymatique, à l'aide d'une saccharose glycosyl-transférase, du saccharose ou glucosyl-α-1,2-fructose.

On pourra se référer, entre autres documents qui concernent l'obtention et les propriétés du palatinitol, à l'ouvrage « Alternative Sweeteners » édité en 1986 par LYN O'BRIEN NABORS, chap. 11, pp. 217-244.

Cependant, ce procédé d'isomérisation ne permet d'obtenir qu'un mélange grossièrement équimolaire de glucosyl-α-1,6-mannitol et de glucosyl-α-1,6-sorbitol (ou isomaltitol), sans qu'il ne soit possible de faire varier la composition relative des deux constituants dans le mélange.

Dans les demandes de brevet WO 97/19093 et WO 97/19093 dont la société Demanderesse est titulaire, il a été montré que le palatinitol pouvait être également préparé à partir de l'isomaltose, ou glucosyl-α-1,6-glucose.

L'isomaltose est quant à lui préparé à partir du glucose ou d'un sirop de maïs comme par exemple décrit dans la demande de brevet FR 2.515.186.

Cependant, si ces procédés alternatifs permettent d'obtenir le palatinitol en s'affranchissant de l'obligation d'utiliser le saccharose comme matière première, il n'en demeure pas moins que ce mélange de glucosyl-α-1,6-mannitol et de glucosyl-α-1,6-sorbitol ne peut toujours être obtenu qu'en proportion équimolaire.

Au mieux, le mélange final peut contenir une proportion élevée en glucosyl-α-1,6-sorbitol, si les techniques de chromatographie normalement mises en oeuvre dans les procédés des demandes de brevets WO 97/19093 et WO 97/19093 pour séparer les composants dudit mélange afin de conduire à l'obtention d'un mélange équimolaire, ne sont pas utilisées.

En outre, l'enrichissement du mélange en isomaltitol peut être avantageusement effectué en réalisant simplement l'ajout dudit composé obtenu par ailleurs par hydrogénation catalytique de l'isomaltose.

De tout ce qui précède, il résulte qu'il existe un besoin non satisfait de disposer d'un procédé simple permettant, en toute généralité, d'obtenir un mélange de glucosyl-mannitol et de glucosyl-sorbitol, quel que soit le type d'isomère de liaison considéré, en proportion au moins équimolaire, et plus particulièrement enrichi en son composant glucosyl-mannitol.

Soucieuse de développer un procédé qui permette de répondre mieux que ceux qui existent déjà aux contraintes de la pratique, la société Demanderesse a constaté que cet objectif pouvait être atteint par un procédé d'hydrogénation catalytique de la glucosyl-glucosone, conduite dans des conditions particulières de température et de pression.

On entend par « glucosone », également connue sous les noms de 2 céto-glucose ou de D-arabino-2-hexulosone, le produit du traitement chimique du glucose à l'eau oxygénée ou avec de l'acétate de cuivre, ou avantageusement le produit de la conversion enzymatique du glucose à l'aide d'une pyranose 2-oxydase.

Il est d'autre part connu dans l'état de la technique que la pyranose 2-oxydase présente encore un large spectre de modification enzymatique.

Ainsi HUWIG et al. montrent dans Med. Fac. Landbouww. Univ. Gent., 62/4a,1997, pp. 1193-1197 que la pyranose-2-oxidase isolée de *Peniophora gigantea* est capable d'agir sur l'isomaltose pour conduire au produit d'oxydation de son glucose en position réductrice terminale, soit la glucosyl-α-1,6-glucosone.

De la même manière, VOLC et al. montrent dans Arch. Microbiol., 1997, 167, pp. 119-125, que la pyranose 2-oxydase isolée *d'Agaricus bisporus* agit sur le maltose pour conduire à la glucosyl-α-1,4-glucosone.

Cependant, nulle part n'est décrit ou suggéré dans ces documents qu'il soit possible d'utiliser avantageusement ces deux isomères de liaisons en α-1,4 et α-1,6 de la glucosyl-glucosone pour obtenir, par un procédé d'hydrogénation particulier, un mélange de glucosyl-mannitol et de glucosyl-sorbitol en proportion au moins équimolaire, et plus particulièrement un mélange contenant une proportion élevée en glucosyl-mannitol, de préférence au moins égal à 1,5.

La société Demanderesse a donc eu le mérite de trouver un procédé de préparation d'un mélange de glucosyl-mannitol et de glucosyl-sorbitol, caractérisé par le fait que l'on conduit l'hydrogénation de la glucosyl-glucosone en présence d'un catalyseur, et en soumettant une solution de glucosyl-glucosone présentant une matière sèche au moins égale à 10 % en poids, de préférence comprise entre 20 et 50 % en poids, à une pression au moins égale à 30 bars, de préférence comprise entre 30 et 200 bars, et à une température au moins égale à 50°C, de préférence comprise entre 50 et 150°C.

Ce faisant, la société Demanderesse a trouvé que le procédé conforme à l'invention permet de conduire indifféremment l'hydrogénation des isomères de liaison α1,4 ou α-1,6 de la glucosyl-glucosone.

Le procédé conforme à l'invention met en oeuvre une solution de glucosyl-glucosone présentant une matière sèche au moins égale à 10 % en poids, de préférence comprise entre 20 et 50 % en poids.

On choisit de préparer la solution à hydrogéner avec de la glucosyl-glucosone de très haute pureté, avantageusement produite par voie enzymatique par tout moyen connu en soi par l'homme du métier, à partir du maltose ou de l'isomaltose. Le choix de l'isomère de liaison α-1,4 ou α-1,6 de la glucosyl-glucosone comme matière première à hydrogéner repose alors sur la nature du mélange de glucosyl-mannitol ou de glucosyl-sorbitol que l'on souhaite obtenir.

On entend par « haute pureté », une teneur en glucosyl-glucosone de l'ordre de 100 %.

Le catalyseur est choisi dans le groupe constitué du palladium, du nickel, du ruthénium, du platine, du rhodium, du cobalt, du cuivre, du zinc, du chrome, du manganèse, du tungstène, et est de préférence du nickel.

Dans un mode préférentiel de réalisation du procédé conforme à l'invention où le catalyseur est placé en suspension dans la solution de glucosyl-glucosone à hydrogéner, on choisit avantageusement le nickel sous sa forme dite Ni de Raney.

Dans un autre mode préférentiel du procédé conforme à l'invention, le catalyseur peut être imprégné ou co-échangé sur un support inerte, de préférence choisi dans le groupe constitué du charbon actif, de la tourbe, des zéolites, des aluminosilicates, du dioxyde de titane. De préférence il est constitué de charbon actif.

Le rapport pondéral catalyseur / support inerte est établi avantageusement à une valeur comprise entre 1 et 5 %, de préférence de l'ordre de 2 %.

Il est également possible de mettre en oeuvre un catalyseur comportant un agent promoteur. Cet agent promoteur peut être choisi dans le groupe constitué du titane, du molybdène et du platine.

Dans un mode de réalisation du procédé conforme à l'invention, on réalise une suspension de glucosyl-glucosone d'une matière sèche comprise entre 10 et 50 %, et introduit le catalyseur sous sa forme Ni de Raney.

On choisit alors de mettre en oeuvre une pression d'hydrogénation au moins égale à 50 bars, de préférence comprise entre 30 et 200 bars, de préférence de l'ordre de 100 bars et une température au moins égale à 50°C, de préférence comprise entre 50 et 150°C, et plus préférentiellement encore une température de l'ordre de 125°C.

Les conditions de pression sont généralement choisie de manière à éviter substantiellement l'hydrolyse, même partielle, de la glucosyl-glucosone en glucose et glucosone, susceptible de conduire à l'apparition de fructose, de sorbitol ou de mannitol dans le mélange final de glucosyl-mannitol et de glucosyl-sorbitol.

Dans un autre mode de réalisation du procédé conforme à l'invention, on choisit de conduire l'hydrogénation en mode continu sur lit fixe de catalyseur.

Le catalyseur est alors disposé dans un lit fixe sous la forme d'un empilement compact de particules, le tout placé sur des grilles de soutien dans un réacteur d'hydrogénation. On choisit avantageusement un réacteur à ruissellement.

Au sens de l'invention, on entend par « réacteur à ruissellement », un réacteur d'hydrogénation dans lequel une phase liquide contenant le produit à hydrogéner et une phase gaz circulent de manière co-courante ou contre-courante, de préférence de manière co-courante de haut en bas, dans un lit fixe de particules de catalyseur où s'effectue la réaction d'hydrogénation.

Les débits d'écoulement de ces deux phases sont réglées pour permettre au liquide de ruisseler sur lesdites particules de catalyseur, et assurer le meilleur contact entre les deux phases liquide et gazeuse d'une part, et la phase solide du catalyseur d'autre part.

Dans un mode de réalisation du procédé conforme à l'invention, on choisit de préparer un lit fixe constitué de 200 l de catalyseur commercial, un débit d'alimentation de la solution de glucosyl-glucosone, d'une matière sèche comprise entre 10 et 50 % en poids, à une valeur comprise entre 150 et 250 kg/h, et une quantité d'hydrogène introduite dans ledit réacteur à ruissellement de l'ordre de deux à quinze fois la stoechiométrie de la réaction.

Les pression et température d'hydrogénation mises en oeuvre sont alors avantageusement choisies à des valeurs respectives de l'ordre de 30 à 150 bars, par exemple 150 bars, et de l'ordre de 50 à 150°C, par exemple 90 °C.

Le mélange de glucosyl-mannitol et de glucosyl-sorbitol est alors obtenu en proportion équimolaire et avec un rendement de conversion au moins égal à 90 %.

Dans un mode préférentiel de réalisation du procédé conforme à l'invention, on choisit de mettre en oeuvre un procédé continu d'hydrogénation de la glucosyl-glucosone dans une succession de lits fixes de catalyseur disposés en série et en au moins deux zones de réaction.

L'hydrogénation est alors avantageusement conduite dans une première zone de réaction, constituée d'au moins un lit fixe de catalyseur, de manière à obtenir une conversion élevée de la glucosyl-glucosone en un mélange de glucosyl-mannose, de glucosyl-fructose et de glucosyl-glucose, contenant une proportion élevée en glucosyl-mannose, puis dans une seconde zone de réaction, constituée d'au moins un lit fixe de catalyseur, où l'hydrogénation est conduite de manière à obtenir le mélange de glucosyl-mannitol et de glucosyl-sorbitol avec un taux de conversion et une proportion en glucosyl-mannitol élevés.

La société Demanderesse a ainsi trouvé qu'il est possible de conduire l'hydrogénation en mode continu et en au moins deux zones de réaction, de manière à séparer l'étape de production du mélange de glucosyl-mannose, de glucosyl-fructose et de glucosyl-glucose de celle de production du mélange de glucosyl-mannitol et de glucosyl-sorbitol, pour obtenir le mélange de glucosyl-mannitol et de glucosyl-sorbitol avec une teneur élevée en glucosyl-mannitol encore jamais atteinte, et avec une productivité et un taux de conversion élevés.

On peut mettre en oeuvre des catalyseurs de nature différente dans chacune des zones de réaction. Cependant, on préfère utiliser un catalyseur de même nature dans ces deux zones.

Dans la première zone de réaction, les conditions de pression sont réglées de manière à obtenir une conversion élevée, au moins égale à 80 % de la glucosyl-glucosone en un mélange de glucosyl-mannose, de glucosyl-fructose et de glucosyl-glucose, dont la proportion en glucosyl-mannose est élevée.

Au sens de l'invention, on entend par « proportion en glucosyl-mannose élevée », une teneur en glucosyl-mannose au moins égale à 40 % en poids, de préférence au moins égale à 70 % en poids du mélange.

La pression d'hydrogénation dans la première zone de réaction est fixée à une valeur au moins égale à 50 bars, comprise entre 50 et 100 bars, de préférence de l'ordre de 80 bars.

Dans la seconde zone de réaction, les conditions de pression sont réglées de manière à obtenir le mélange de glucosyl-mannitol et de glucosyl-sorbitol, contenant le glucosyl-mannitol en proportion élevée, avec un taux de conversion au moins égal à 95 %.

Au sens de l'invention, on entend par « proportion élevée en glucosyl-mannitol , un rapport pondéral glucosyl-mannitol sur glucosyl-sorbitol au moins égal à 1,5.

La pression d'hydrogénation dans la seconde zone de réaction est fixée à une valeur au moins égale à 100 bars, comprise entre 100 et 200 bars, de préférence de l'ordre de 150 bars.

De préférence, la température d'hydrogénation est maintenue constante dans les deux zones de réaction à une valeur comprise entre 50 et 100°C, de l'ordre de 90°C comme exemplifié ci-après.

Les lits fixes de catalyseur sont avantageusement disposés dans des réacteurs à ruissellement. On choisit de mettre en oeuvre 200 l de particules de catalyseur commercial, un débit d'alimentation de la solution de glucosyl-glucosone d'une matière sèche comprise entre 10 et 50 % en poids à une valeur comprise entre 200 et 300 kg/h, et une quantité d'hydrogène comprise entre deux et quinze fois la stoechiométrie de la réaction.

D'autres caractéristiques et avantages de l'invention apparaîtront à la lecture des exemples non limitatifs décrits ci-dessous.

### Exemple 1

La réaction d'hydrogénation est effectuée dans un seul réacteur à ruissellement renfermant un unique lit fixe de catalyseur au nickel sur charbon, dans lequel on fait circuler l'hydrogène et la solution de glucosyl-α-1-6-glucosone de manière co-courante du haut vers le bas dudit réacteur.

Le lit fixe de catalyseur au nickel est constitué d'un empilement compact de grains de catalyseur cylindriques.

Chaque grain de catalyseur est composé de charbon actif de type NORIT RX08, sous la forme d'un cylindre de 0,8 mm de diamètre et de 1 à 5 mm de longueur, contenant 2 % en poids de nickel.

Le réacteur contient de l'ordre de 200 l de catalyseur, disposé en lit fixe de 30 cm de diamètre et de l'ordre de 3 m de hauteur.

On alimente simultanément le réacteur avec une solution de glucosyl-glucosone à 30 % de matière sèche à un débit de 200 kg/h, et de l'hydrogène à raison de 12 kg/h.

La pression de fonctionnement dans le réacteur est de 150 bars, et la température de 110 °C.

En sortie de réacteur, le taux de conversion obtenu est de 92,5 %, et le rapport de glucosyl-α-1,6-mannitol et de glucosyl-α-1,6-sorbitol dans le mélange est équimolaire.

### Exemple 2

La réaction d'hydrogénation est effectuée dans deux réacteurs à ruissellement connectés en série avec un échangeur thermique intermédiaire.

Les deux réacteurs renferment chacun un unique lit fixe de catalyseur au nickel sur charbon, dans lequel on fait circuler l'hydrogène et la solution de glucosyl-α-1-4-glucosone de manière co-courante du haut vers le bas desdits réacteurs.

Chaque lit fixe de catalyseur au nickel est constitué d'un empilement compact de grains de catalyseur cylindriques.

Chaque grain de catalyseur est composé de charbon actif de type NORIT RX08, sous la forme d'un cylindre de 0,8 mm de diamètre et de 1 à 5 mm de longueur, contenant 2% en poids de nickel.

Chaque réacteur contient de l'ordre de 200 l de catalyseur, disposé en lit fixe de 30 cm de diamètre et de l'ordre de 3 m de hauteur.

On alimente simultanément la première zone d'hydrogénation avec une solution de glucosyl-glucosone à 30 % de matière sèche, à un débit de 250 kg/h et de l'hydrogène à raison de 12 kg/h.

La pression de fonctionnement dans le premier réacteur est de 80 bars, et la température de 90 °C.

La température d'entrée dans le second réacteur est maintenue à 90°C, et la pression fixée à 150 bars.

A la sortie du premier réacteur, le taux de conversion est de 75 %, et le mélange de glucosyl-α-1,4-mannose, de glucosyl-α-1,4-fructose et de glucosyl-α-1,4-glucose présente la composition suivante : 65 % en poids de glucosyl-α-1,4-mannose, 15 % de glucosyl-α-1,4-fructose, et 20 % de glucosyl-α-1,4-glucose.

A la sortie du second réacteur, la conversion est de 98 %, et le mélange de glucosyl-α-1,4-mannitol et de glucosyl-α-1,4-sorbitol présente un rapport de glucosyl-α-1,4-mannitol sur glucosyl-α-1,4-sorbitol de 2,5.

## Revendications

1. Procédé de préparation d'un mélange de glucosyl-mannitol et de glucosyl-sorbitol, caractérisé par le fait que l'on conduit l'hydrogénation de la glucosyl-glucosone en présence d'un catalyseur, et en soumettant une solution de glucosyl-glucosone présentant une matière sèche au moins égale à 10 % en poids, de préférence comprise entre 20 et 50 % en poids, à une pression au moins égale à 30 bars, de préférence comprise entre 30 et 200 bars, et à une température comprise entre au moins égale à 50°C, de préférence comprise entre 50 et 150°C.

2. Procédé selon la revendication 1, caractérisé par le fait que le catalyseur est choisi dans le groupe constitué du palladium, du nickel, du ruthénium, du platine, du rhodium, du cobalt, du cuivre, du zinc, du chrome, du manganèse, du tungstène, et est de préférence du nickel.

3. Procédé selon l'une ou l'autre des revendications 1 et 2, caractérisé par le fait que l'on conduit l'hydrogénation de la glucosyl-glucosone en mode continu sur lit(s) fixe(s) de catalyseur.

4. Procédé selon la revendication 3, caractérisé par le fait que l'hydrogénation continue est conduite sur au moins un lit fixe de catalyseur dans lequel la pression est comprise entre 30 et 150 bars et la température est comprise entre 50 et 150°C.

5. Procédé selon l'une ou l'autre des revendications 3 et 4, caractérisé par le fait que l'on conduit l'hydrogénation continue de la glucosyl-glucosone dans une succession de lits fixes de catalyseur disposés en série qui comprend :
- une première zone d'hydrogénation, constituée d'au moins un lit fixe de catalyseur, où la pression est au moins égale à 50 bars, de préférence comprise entre 50 et 100 bars,
- une seconde zone d'hydrogénation, constituée d'au moins un lit fixe de catalyseur, où la pression est au moins égale à 100 bars, de préférence comprise entre 100 et 200 bars.

6. Procédé selon la revendication 5, caractérisé par le fait que la température d'hydrogénation dans les deux zones de réaction est comprise entre 50 et 100°C.

7. Procédé selon l'une quelconque des revendications 1 à 6, caractérisé par le fait que la glucosyl-glucosone est préférentiellement choisie dans le groupe des isomères de liaison de la glucosyl-glucosone constitué de la glucosyl-α-1,4-glucosone et de la glucosyl-α-1,6-glucososone.

8. Mélange de glucosyl-α-1,4-mannitol et de glucosyl-α-1,4-sorbitol dans un rapport glucosyl-α-1,4-mannitol sur glucosyl-α-1,4-sorbitol au moins égal à 1, de préférence au moins égal à 1,5, susceptible d'être obtenu par la mise en oeuvre d'un procédé conforme à l'une quelconque des revendications 1 à 7.
